# EUROPEAN PATENT APPLICATION

(11) **EP 1 142 910 A1**
(43) Date of publication of application: **10.10.2001**
(21) Application number: 00107515.9
(22) Date of filing: 07.04.2000
(51) Int. Cl.: C07K 16/40, A61K 39/395, A61K 31/7088, A61P 37/00

(54) **Inhibitors for the formation of soluble human CD23**

(71) Applicant: Frey, Jürgen, Prof. Dr., 33617 Bielefeld (DE)
(72) Inventor: Frey, Jürgen, Prof. Dr., 33617 Bielefeld (DE)
(74) Representative: Meyer-Dulheuer, Karl-Hermann, Dr.

(57) **Abstract**

A pharmaceutical composition for the treatment or prophylaxis of disorders is described in which the overproduction of sCD23 is implicated. This composition comprises an inhibitor for the formation of human soluble CD23 which inhibitor decreases or blocks selectively the activity of the metalloprotease ADAM9 which otherwise mediates the shedding of sCD23 in human B-cell lines. Also described is a pharmaceutical composition wherein the inhibitor for the formation of human soluble CD23 is a monoclonal or polyclonal antibody directed against the metalloprotease ADAM9 or wherein the inhibitor is an antisense oligonucleotide which is specific for c-myc. Such a pharmaceutical composition may be used in a method for selectively inhibiting the formation of ADAM9 as well as the formation of sCD23. It is a suitable medicament against inflammatory disorders, autoimmune diseases and allergy.

## Description

This invention relates to inhibitors for the formation of soluble human CD23 and for the treatment of conditions associated with excess production of soluble CD23 (s-CD23) such as autoimmune diseases, inflammatory processes and allergy.

Matrix metalloproteases such as collagenase, stromelysin and gelatinase are known to be involved in connective tissue breakdown. Known classes of matrix metalloprotease inhibitors include derivatives of hydroxamic acid, barbituric acid, phosphonates and thiols.

International patent application WO 93/20047 discloses that inhibitors of the matrix metalloproteases, especially derivatives of hydroxamic acid, are potentially useful for the treatment of prophylaxis of conditions involving such tissue breakdown, for example rheumatoid arthritis, osteopenias such as osteoporosis, periodontitis, gingivitis, corneal epidermal or gastric ulceration, and tumor metastasis or invasion.

The low affinity receptor for IgE, FcεRII (CD23), is a type two membrane glycoprotein belonging to the C-type (calcium dependent) lectin family (Kikutani et al., 1986). Some members of this family have been shown to be adhesion molecules. The lectin domain of CD23 comprises the IgE binding site (Bettler et al., 1989). IgE binding is a calcium dependent process (Drickamer et al., 1988; Richards et al., 1990). CD23 is expressed on a variety of haemopoietic cell types such as B and T lymphocytes, a subset of thymic epithelial cells, monocytes, macrophages, follicular dendritic cells (FDC), platelets, eosinophils, Epstein-Barr virus (EBV)-containing nasopharyngeal carcinoma cells, Langerhans cells and natural killer (NK) cells (Delespesse et al., 1992). Human CD23⁺ cells can express two different forms of CD23, CD23a and CD23b (Yokota et al., 1988) which differ only at the N-terminal cytoplasmic region (7 aa) whereas the C-terminal extracellular region is identical. Both forms are derived from one gene by utilizing different transcriptional initiation sites and alternative RNA splicing. CD23a is constitutively expressed only on mature B cells (IgM⁺/IgD⁺) in the periphery and B-cell lines, whereas IgM⁺/IgD⁺ B cells in the bone marrow (BM) are CD23⁺. B-cells in the follicular mantle of tonsills are CD23⁺, those in the germinal centre are CD23⁺. The expression of CD23 can be strongly induced by interleukin 4 (IL-4), known to induce germline IgE transcription (Gordon, J. et al., 1991; Delespesse, G. et al., 1992), but is lost after isotype-switching to IgG, IgA and IgE. In some malignant pre-B cells from acute lymphoblastic leukemia patients CD23 expression can also be induced by IL-4 (Law, C.L. et al., 1991). Signals delivered via CD40 on B cells (Clark, 1990) strongly potentiate the IL-4 induced induction of CD23 on mature B cells. This second signal is provided by physical interaction of B-cells with T cells expressing CD40 ligand (CD40L) (Bonnefoy et al., 1996). Furthermore, IL-13 and IL-4, both known to increase CD23 expression, also induce the production of IgE in normal B-cells due to isotype switching (Punnonen et al., 1993; Lebman et al., 1988). Factors counteracting the IL-4 induced CD23 expression (INF-γ, INF-α and PGE2) also blocked the IgE synthesis by B-cells (Pene et al., 1988; Defrance et al., 1987).

CD23b is mainly found on activated monocytes, macrophages, eosinophils, dendritic cells, platelets, and transiently on IL-4 treated B-cells (Delespesse 1992; Munoz et al., 1998). Ligation of CD23 on human monocytes triggers monokine release (Bonnefoy et al., 1996). Monocyte activation can be regulated by the specific interaction of CD23 with the α chains of the β2 integrin adhesion molecule complexes CD11b-CD18 and CD11c-CD18 causing an increase in nitrogen oxide (NO) and oxidative product (H₂O₂) as well as proinflammatory cytokines (IL-1β, IL-6, and TNFα; Lecoanet-Henchoz et al., 1995). Increased levels of CD23 are found in different chronic inflammatory diseases such as RA (Hellen et al., 1991), SLE (Bansal et al., 1992) and glomerulonephritis (Yano et al., 1992). Consistent with these findings are results obtained for CD23 in collagen-induced arthritis (CIA) in mice, a model for RA. The percentage of CD23⁺ lymph node cells increased after collagen type II immunization. In contrast, CD23-deficient mice developed CIA with a delayed onset and reduced severity (Kleinau et al., 1999).

Transient induction of CD23 by IL-4 on the plasma membrane of B-cells and monocytes is accompanied by concomitant proteolytic release (shedding) of different defined soluble CD23 (sCD23) fragments (Letellier et al., 1990; Letellier et al., 1989). Different cytokine activities have been attributed especially to the soluble 25 kDa form of CD23. sCD23 has been shown to act as an autocrine growth factor in some EBV transformed B-cells lines (Swendeman et al., 1987) and as a differentiation promoting factor for prothymocytes (Mossalayi et al., 1990). Furthermore, sCD23 can prevent apoptosis of germinal centre B-cells, most likely via induction of bcl-2 induction (Liu et al., 1991a; Liu et al., 1991b). It has been shown recently, that the vitronectin receptor (VnR), αᵥβ₃ integrin, which binds to several ligands, may also be a functional receptor for sCD23 on monocytes and macrophages (Hermann et al., 1999).

The mechanisms by which soluble CD23 fragments are generated have not been well characterized. Batimastat as well as a number of other hydroxamic acid-based inhibitors of metalloproteinases, inhibit proteolytic processing of CD23 in nanomolar concentrations (Christie et al., 1997, Wheeler et al., 1998, Bailey et al., 1998a; Balley et al., 1998b). In a more recent attempt to characterize the proteinase involved in CD23 shedding a CD23 processing activity was enriched by gel chromatography from plasma membrane fractions of the human B-cell line RPMI8866. (Marolewski et al., 1998). The ectodomain shedding of membrane proteins shows some common features. In most of these cases proteolytic release can be blocked by hydroxamic acid-based inhibitors, processing occurs at a fixed distance from the plasma membrane, and shedding can be induced by activation of protein kinase C (PKC) (Hooper et al., 1997). The shedding of pro-TNF-α by TACE is the first example showing that a metalloproteinase is involved (Black et al., 1997; Moss et al., 1997). TACE is a member of the growing ADAM or MDC family (for a review: Blobel and White, 1992; Black and White, 1998; Schlondorff and Blobel, 1999) all having a conserved metalloprotease domain, but only 15 (out of 28) are supposed to be active metalloproteases because of the highly conserved consensus sequence (HEXXH) which is part of the catalytic domain (Bode et al., 1993).

It has now been found that the metalloprotease ADAM9 which is widely expressed (Weskamp et al., 1996), is involved in shedding human CD23b. It could be demonstrated that ADAM9, but not ADAM8, ADAM15 and ADAM19, expressed in either COS or B-cells leads to a significant reduction of surface bound CD23b coexpressed in these cells. Human B-cell lines overexpressing ADAM9 showed a marked reduction in surface CD23. In addition it could be shown that upregulation of c-myc in human B-cells leads to a significant increase in ADAM9 expression accompanied by increased shedding (40%) of CD23, i.e. production of sCD23.

These results led to the first subject of the present invention which is a pharmaceutical composition for the treatment or prophylaxis of disorders in which the overproduction of s-CD23 is implicated, which comprises an inhibitor for the formation of human soluble CD23, wherein the inhibitor is a compound which selectively decreases or blocks the activity of the metalloprotease ADAM9 which otherwise mediates the shedding of s-CD23 in human B-cells as well as all other CD23 positive cells all of which also express ADAM9.

A further subject of the invention is a pharmaceutical composition , wherein the inhibitor for said ADAM-protein is a monoclonal or polyclonal antibody which is selectively directed against the metalloprotease ADAM9, or is a synthetic inhibitor from the group of hydroxamic acid based or barbituric acid based inhibitors which selectively decreases or blocks the activity of the metalloprotease ADAM9.

A further subject of the invention is a pharmaceutical composition which comprises an antisense oligonucleotide which is specific for c-myc.

Further subjects of the invention are antibodies which selectively bind to the metalloprotease ADAM9 and their use in the manufacture of medicaments which are useful for the treatment of patients suffering from disorders which are associated with an overproduction of the soluble s-CD23.

Finally, the invention is directed to the use of antibodies and of oligonucleotides for the treatment of said patient.

From the above-mentioned findings it is obvious that ADAM9 is involved in the formation of sCD23 and that by the inhibition or blocking of ADAM9 the amount of sCD23 may be drastically reduced. This aim can be achieved on several ways:

A first successful route for the inhibition of ADAM9 consists in the production of monoclonal or polyconal antibodies which are selectively directed against said metallo-protease. Such antibodies have been generated by known methods in mice. The murine antibodies have lateron been humanised. They proved to be valuable and selctive inhibitors of ADAM9 and could be used for the manufacture of a medicament for the treatment of disorders which are associated with overproduction of soluble human CD23 by administering a therapeutically effective amount of said antibody.

A second successful route for the inhibition of ADAM9 is based on antisense technology. Antisense technology is emerging as an effective means of lowering the levels of a specific gene product. It is based on the findings that these "antisense" sequences hybridize a gene or associated target polynucleotide, to form a stable duplex or triplex, based upon Watson-Crick or Hoogsteen binding, respectively. The specifically bound antisense compound then either renders the respective targets more susceptible to enzymatic degradation, blocks translation or processing, or otherwise blocks or inhibits the function of a target polynucleotide. Where the target polynucleotide is RNA, the antisense molecule hybridizes to specific RNA transcripts disrupting normal RNA processing, stability, and translation, thereby preventing the expression of a targeted gene. Administration of antisense oligonucleotides or transfer of expression constructs capable of producing intracellular antisense sequences complementary to the mRNA of interest have been shown to block the translation of specific genes in vitro and in vivo. For example, Holt et al., Mol. Cell. Biol. 1988, 8 963-973, focusing on c-myc, found the formation of an intracellular duplex with target mRNA and a selective decrease of c-myc protein in human promyelocytic leukemia HL-60 cells.

According to the present invention the shedding of CD23 can not only be blocked by the inhibition of ADAM9 but also by means which are suitable to inhibit c-myc. This can be achieved by an antisense oligonucleotide specific for c-myc. Therefore the present invention provides a method for inhibiting the generation of ADAM9 by administering to a patient suffering from overproduction of sCD23 an antisense oligonucleotide which is complementary to a region of mRNA encoding c-myc. Such antisense oligonucleotide may be used in the manufacture of a medicament by which therapeutically effective amounts are administered to a patient who needs a reduction of the level of sCD23 in his organism.

These findings are the result of the following experiments:

### Overexpression of ADAM9 results in ectodomain shedding of CD23b in COS-7 cells

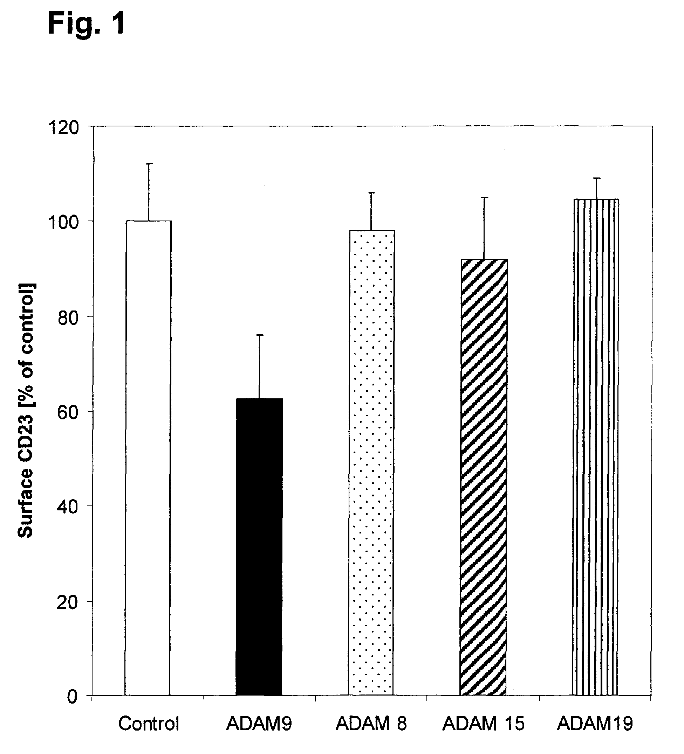

To analyse whether different ADAMs are involved in ectodomain shedding of CD23, first the effect of ADAM8, ADAM9, ADAM15 and ADAM19 expression on CD23b in COS-7 cells was tested. Fourty-eight hours after cotransfection of full length CD23b with either ADAM8, 9, 15, 19 or vector alone surface bound CD23b was analyzed by FACS analyses. Among the ADAMs tested, only ADAM9 coexpression reduced the number of surface bound CD23b significantly (35-40%). A minor effect of ADAM15 (5-10%) on CD23 shedding was observed, whereas neither ADAM8 nor ADAM19 coexpression did alter the number of surface CD23 molecules (Fig. 1). Cotransfection with the respective expression vectors (pcDNA3 or pTOP) did not influence the surface expression of CD23 in COS cells. Thus, ectopic overexpression of ADAM9 and to a minor extend ADAM15 results in the ectodomain processing of CD23.

### Upregulation of ADAM9 in CHO cells leads to the Generation of sCD23

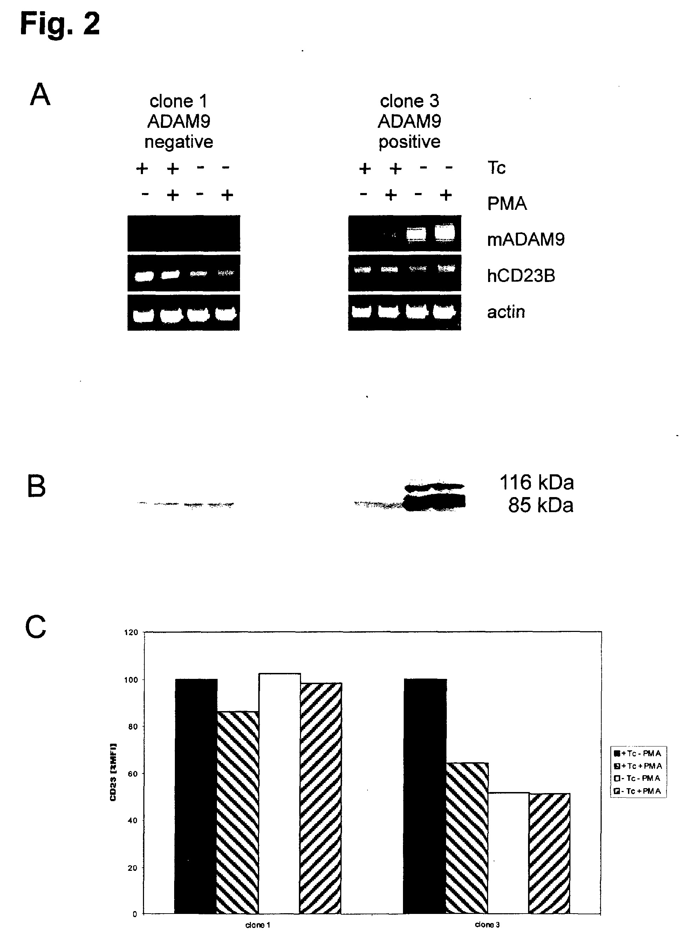

To exclude the possibility that the transient overexpression of ADAM9 in COS cells leads to the release of other metalloproteinases which might be responsible for the observed shedding, stable CHO clones were generated which constitutively express CD23 and allow to turn on ADAM9 transcription by withdrawing tetracycline (TC) from the medium (CHOAA8 cells). All selected clones grown in the absence or presence of TC were first analysed for CD23 and ADAM9 expression by RT-PCR. Fig. 2 shows clone 1, where the integration of the ADAM9 cDNA under the control of the CMVₘᵢₙ, promotor is in a chromosomal surrounding that makes it unresponsive to TC regulation (this clone was used in all further experiments as a negative control). The PCR data clearly show that also in the absence of TC there is no ADAM9 transcript detectable using 30 PCR cycles (Fig. 2A). In contrast, CD23b and actin transcripts could be easily detected after 20 PCR cycles. In Western blot analyses of whole cell lysates with anti-AD9Dis only faint bands of Mᵣ 85kDa could be detected (Fig. 2B). Because no ADAM9 transcript in RT-PCR (30 cycles) could be detected it is likely that the faint 85kDa bands are due to crossreaction of the anti-AD9Dis antibody with endogenous ADAM9. Also stimulation of PKC activity by adding PMA did not alter the expression of ADAM9. In several other clones tested the ADAM9 cDNA under the control of the CMVₘᵢₙ, promotor had integrated into chromosomal loci allowing TC dependent expression of ADAM9. Clone 3 gave the best signal to background ration among all clones tested. In the presence of TC only a comparably low background level of ADAM9 transcript could be found. After stimulation of the cells with PMA a slight increase in ADAM9 transcript could be observed. (Fig. 2A). In contrast, the transcript levels of constitutively expressed CD23b as well as actin were neither altered by TC nor by PMA treatment (Fig. 2A). Removal of TC from the cells leads to strong increase in ADAM9 transcript that could be slightly increased by PMA treatment of the CHO cells (Fig. 2A). The observed induction in ADAM9 mRNA leads to the dramatic increase in ADAM9 protein synthesis as judged by Western blotting using anti-AD9Dis antibodies. Besides the mature 85kDa form also some unprocessed protein with a Mᵣ of 116 kDa could be detected. The ratio of both forms was not significantly altered by PMA treatment (Fig. 2B). The effect of PMA treatment as well as the upregulation of ADAM9 on surface bound CD23b was analyzed with clone 3 and compared with the data obtained with clone 1 as a negative control (Fig. 2C). Surface bound CD23b in the presence of TC (repression of ADAM9 expression) was considered as 100% of CD23b. With clone 1 no significant alteration in surface bound CD23b after PMA treatment of the cells could be seen either in the presence or absence of TC. Always a slight decrease (10%) in surface bound CD23b after PMA stimulation in the presence of TC was observed. In contrast, PMA treatment of clone 3 in the presence of TC (ADAM9 expression repressed) resulted in an approx. 30% reduction of surface bound CD23b. Obviously, the comparably low amount of ADAM9 already expressed in the presence of TC due to leakyness of the system is already sufficient to allow shedding of CD23b to this extent. After upregulation of ADAM9 by withdrawal of TC about 50% of the otherwise surface bound CD23b was removed by shedding. Under these conditions the amount of CD23b removed from the cell surface (50%) could not be further increased by concomittant PMA treatment.

### ADAM9 but not ADAM8, ADAM15 and ADAM19 mediate shedding of endogenous CD23 in human B-cell lines

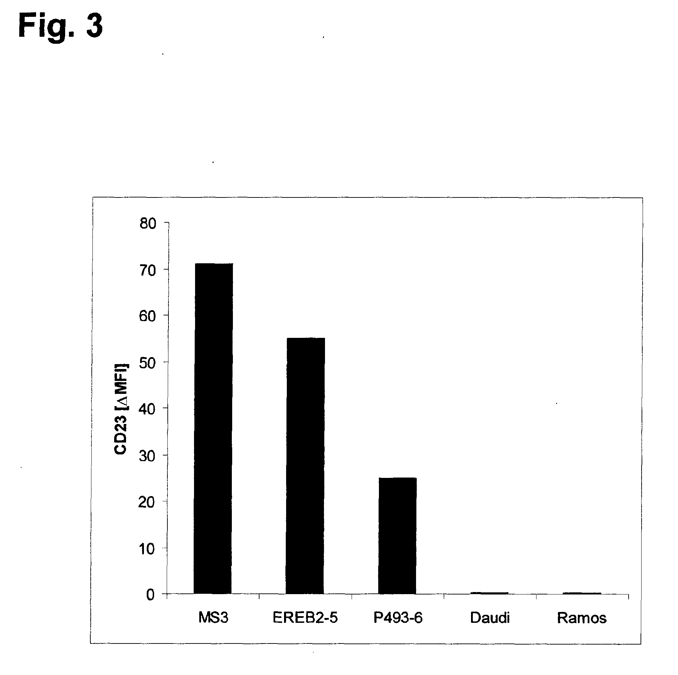

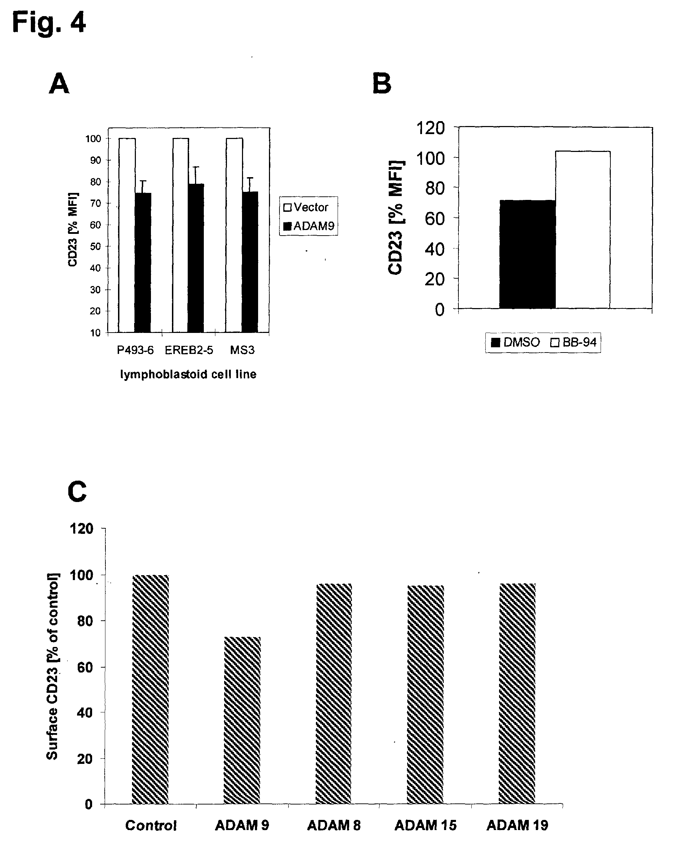

The human B-lymphoblastoid cell lines P493-6 (LCL phenotype showing some features of the BL phenotype after upregulation of c-myc), EREB2-5 (LCL phenotype) and MS3 (EBV immortalized; LCL phenotype) all express endogeneous CD23. FACS analyses with FITC-labelled anti-CD23 showed that MS3 cells and EREB2-5 cells express significantly higher amounts of CD23 compared to P493-6 cells whereas the Burkitt lymphoma cells Daudi and Ramos are CD23 negativ (Fig. 3). The ADAM9 expression level has been further analyzed by RT-PCR using primers specific for human ADAM9 and for actin (Tab. 1). The separated DNA fragments, separated by agarose gel electrophoresis were stained with ethidium bromide. The ADAM9 band intensities, normalized to actin, revealed that MS3 cells, showing the highest level of surface CD23, express the lowest level of ADAM9. To study the influence of ADAM9 in human B-cells on CD23 shedding, ADAM9 cDNA in pcDNA3 or pcDNA3 alone (vector control) was cotransfected with E-GFP (E-GFP-pIRES) into P493-6 cells, EREB2-5 cells and MS3 cells. The number of surface CD23 molecules was analyzed by FACS analysis by gating the E-GFP positive cells (transfected cells). Cells transfected with E-GFP-pIRES served as a control (100% CD23). The influence of the various ADAMs on surface bound CD23 in the respective B--cell lines was analyzed by FACS analyses by gating the E-GFP positive cells. Surface bound CD23 on each cell line tested transfected with E-GFP-pIRES alone served as a control (100% CD23). In all three B-cell lines tested expression of ADAM9 reduced the amount of surface bound CD23 by 25 - 30% compared to controls (Fig.4A). The action of ADAM9 could be completely blocked by adding batimastat (BB-94, a potent metalloproteinase inhibitor) to ADAM9 transfected cells MS3 cells (Fig. 4B). To verify the specificity of ADAM9 mediated shedding in human B-cells we analyzed whether other members of the ADAM family with potential metalloproteinase activity ADAM8, ADAM15, and ADAM19) can also shed endogeneous CD23 in the human B-cell line MS3. In contrast to ADAM9 (see Fig.4A and Fig.4C), overexpression of ADAM8, ADAM15 or ADAM19 in MS3 cells did not significantly impair the level of surface CD23 (Fig. 4C). Thus, the effect of ADAM9 expression on the CD23b level observed in the otherwise CD23 negative CHOAA8 cell line and COS-7 cells was confirmed in human B-cells expressing endogeneous CD23 as well as ADAM9. Furthermore, these results clearly show that ADAM8, ADAM15 and ADAM19 do not contribute to the shedding of CD23b.

### Shedding of CD23 can be induced by ADAM9 induced by upregulation of c-myc in human P493-6 B-cells

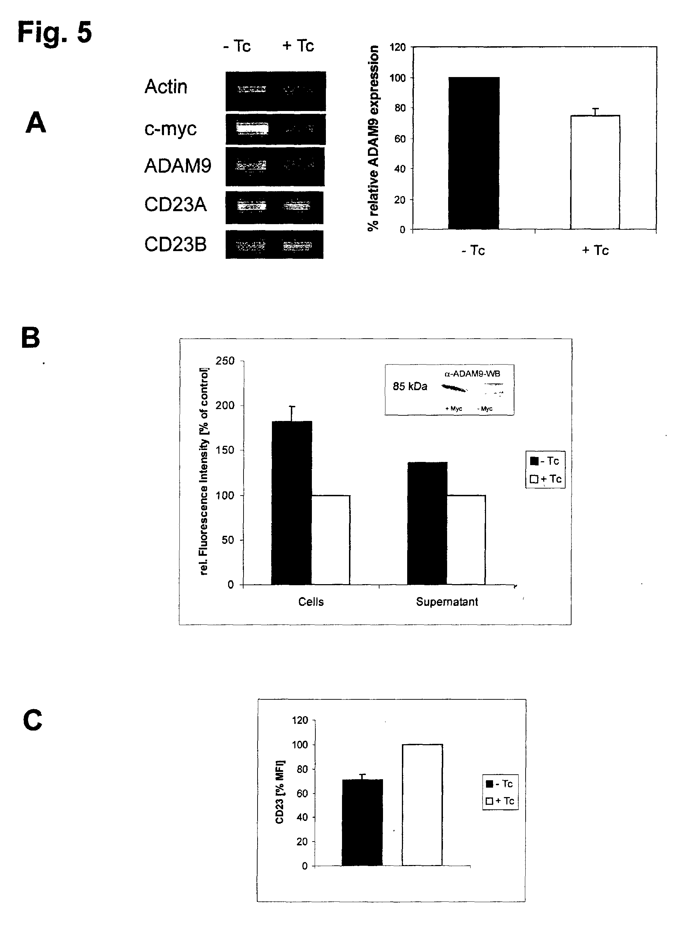

P493-6 cells derived from the conditionally EBV-transformed parental cell line EREB2-5, by transfection with a tetracylin regulatable c-myc (Tet off) (Kempkes et al., 1995) were used to analyze whether changes in c-myc levels alter the number of surface CD23. P493-6 cells grown in the presence or absence of TC were analyzed for the mRNA levels of c-myc, ADAM9, CD23a, CD23b and actin by RT-PCR using specific primers (Tab. 1). Fig. 5A shows that upregulation of c-myc (withdrawal of TC) upregulated the c-myc mRNA level about 4 fold. c-myc upregulation also led to a 60 - 70% increase in CD23a transcript which is constitutively expressed in B-cells whereas the level of CD23b was not affected. In contrast, the level of actin mRNA was found to be constant within the error level. Most interestingly, the level of ADAM9 transcript increased by about 25 - 30% after upregulation of c-myc.

It was then analyzed whether the membrane associated as well as secreted proteolytic activity towards the fluorogenic Mca-peptide (7-methoxycumarin-4-yl)Acetyl-Pro-Leu-Gly-Leu-(3-[2,4-dinitrophenyl]-L-2,3-diamino-propionyl)-Ala-Arg-NH₂changed after upregulation of c-myc. A 70 - 80% increase in Mca-peptide cleavage using the the c-myc upregulated P493-6 cells was found whereas the proteolytic activity released into the medium only increased by 25 - 30% (Fig. 5B).

It is noteworthy that the overall proteolytic activity in the supernatants was very low compared to the membrane associated metalloproteinase activity. Western blot analyses of the ADAM9 protein levels showed that upregulation of c-myc clearly leads to an increase in ADAM9 protein synthesis. The mature 85 kDa form increased 2 - 3 fold upon removal of TC from the culture medium (Fig.5B insert). Thus, at least in this cell system upregulation of c-myc which functions as a transcription factor for several target genes turns on the transcription of the ADAM9 gene. The c-myc induced upregulation of ADAM9 modulated the level of surface CD23 significantly. Comparing membrane bound CD23 on P493-6 cells grown in the presence (100% surface CD23) or absence of TC (upregulation of c-myc and ADAM9) it was found that upregulation via c-myc induction resulted in 35% reduction of CD23 on the cell surface (Fig. 5C).

### c-myc mediated upregulation of ADAM9 releases sCD23

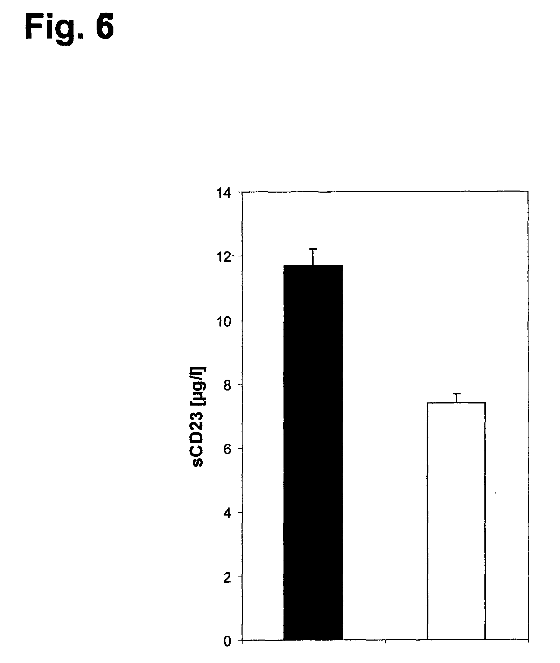

To ensure that the observed reduction on surface CD23 after c-myc mediated induction of ADAM9 is due to increased shedding of CD23, the amount of sCD23 in the culture supernatants of uninduced and induced P493-6 cells was measured quantitatively using a commercially available ELISA assay. It was found that the amount of shedded CD23 was 40 - 50% increased upon upregulation of ADAM9 (Fig.6). These results clearly show that the observed reduction of surface CD23 after c-myc mediated upregulation of ADAM9 is not due to altered CD23 expression. The increased amount of ADAM9 protein correlates with the increase in release of CD23 from the membrane, i.e. production of sCD23 by shedding. The results in transfected COS cells, CHO cells as well as those obtained in the P493-6 cells clearly show that ADAM9 mediates the shedding of CD23 generating the potent proinflammatory mediator sCD23.

### Batimastat but not TIMP-1 or TIMP-2 inhibit CD23 release from P493-6 cells

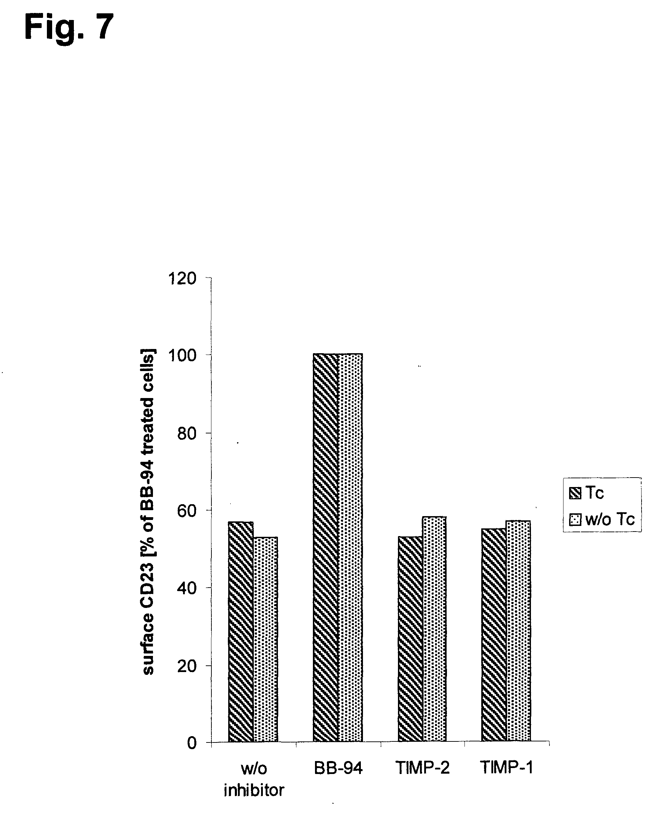

To further characterize the proteolytic activity responsible for the release of CD23 from P493-6 cells the effect of different well known inhibitors of matrix metalloproteinases on the CD23 release from P493-6 cells was tested. These cells constitutively release CD23 from the cell surface which can be increased by upregulation of ADAM9. Having shown that upregulation of ADAM9 leads to an about 25-30% reduction of surface CD23 the effect of batimastat, a hydroxamic acid based inhibitor of matrix metalloproteinases (MMPs) having a broad specificity including ADAM9, was compared with the effect of the endogenous inhibitors of MMPs, TIMP-1 and TIMP-2, which together are known to inhibit all known MMPs on ectodomain shedding of CD23. Culturing P493-6 cells in the presence of batimastat for 48 hours leads to an increase in the number of surface CD23 molecules by 40-50% due to blocking the sheddase activity (Fig. 7). Increased shedding of CD23 upon upregulation of ADAM9 via c-myc activation was also completely blocked by batimastat (40-50% increase in surface CD23; Fig. 7). In contrast, the number of CD23 molecules on P493-6 cells grown in the presence or absence of TC was neither influenced by culturing the cells in the presence of TIMP-1 or TIMP-2. These results clearly show that the sheddase is a metalloproteinase which can be inhibited by batimastat which has been recently shown to inhibit ADAM9 at nanomolar concentrations (Roghani et al. 1999). The finding that neither TIMP-1 nor TIMP-2 block the CD23 sheddase shows that none of the known MMPs, including the membrane-type matrix metalloproteinases (MT-MMPs), can be responsible for the generation of sCD23. These inhibitor studies thus further support the data that ADAM9 is at least the major activity responsible for CD23 shedding.

### Experiments

### Cell Lines and Cell Culture

COS-7 cells were cultured in DMEM supplemented with 10% fetal calf serum, 2mM glutamine, 2mM pyruvate, and essentiell as well as non-essentiell amino acids (Gibco BRL). The cells were split 1:3 the day before transfection.

CHO-AA8-tet-off cells (Clontech), containing the TC regulatable transactivator (Gossen and Bujard, 1992) were cultured as COS cells (see above). The expression of the transactivator was repressed by adding *1m*g/ml TC to the medium. The human B-cell lines Ramos (ATCC CRL 1596), Daudi (ATCC CCL 213), MS3 (EBV immortalized human B-cell line, Staege et al., 2000), EREB2-5 cells (human LCL-, Kempkes et al., 1995), and P493-6 (derived by transfection of EREB2-5 cells with a regulatable c-myc and switching off function of EBV-driven transformation; Schuhmacher et al., 1999) were cultured in RPMI1640 supplemented with 10% FCS and 1*m*M *b*-estradiol (Sigma). To switch off c-myc expression in P493-6 cells 1 *m*g TC (Sigma) was added to the culture medium.

### cDNA Constructs

The full length cDNA of human CD23b was cloned into the expression vectors pcDNA3 (Invitrogen) and pBEHpac18 (Artelt et al., 1988). cDNAs for murine ADAM9, human ADAM15 and mouse ADAM19 were cloned into pcDNA3 (Invitrogen). The cDNA for human ADAM8 was cloned into the expression vector pTOP (Promega). For TC-regulatable expression of ADAM9 the cDNA was cloned into pBI (Clontech). For transient expression studies in the different human B-cell lines (P493-6, MS3, and EREB2-5), the CD23b cDNA cloned into pcDNA3 (CD23b-pcDNA3) was cotransfected with the E-GFP cDNA in pCDM8 (pEGFP-CDM8) or pIRES (pEGFP-pIRES). Positive cells were gated for GFP expression in FACS analyses.

### Transfection of cells.

COS-7 cells (5-6 x 10⁵ cells /10 cm dish) were cotransfected with human CD23 in pcDNA3 (*5m*g) and either ADAM8 (in pTOP)or ADAM9, ADAM15 or ADAM19 in pcDNA3 (7.5*m*g each) using the DEAE Dextran method. Control transfections were performed with the respective expression vectors alone. 16h after transfection the cells were trypsinized and seeded into new plates. 48h after transfection the cells were detached from the plates with cold PBS+2mM CaCl₂ and analysed for CD23 expression by FACS analysis.

The human B-cell lines used were split 1:2 the day before transfection and seeded at a density of 3 - 5 x 10⁵ cells/ml. 10⁷ serum-free washed cells were suspended in 250 *m*l RPMI1640 medium w/o serum. After the addition of the respective plasmid DNA (*20m*g of the respective expression construct or vector control DNA) the samples where chilled on ice for 5 min. Electroporation was performed at 250V, 960 *m*F using the BioRad Gene Pulser device. The pulsed cells were resuspended in 10 ml of culture medium (see above) supplemented with 40 *m*g/ml of gentamycin. To inhibit matrix metalloproteinase activity aliquots of the cells were cultured in the presence of 2 *m*M batimastat (BB-94; British Biotechnology).

### Influence of metalloproteinase inhibitors on CD23 shedding

To inhibit matrix metalloproteinase activity aliquots of P493-6 cells were washed three-times in serum free medium and further cultured either in the presence (*1m*M TC) or absence of TC for 24-48 h either in the presence of 2*µ*M batimastat BB-94; British Biotechnology), 20nM TIMP-1 or 20nM TIMP-2. Control cells where further kept in the presence (*1mM*) or absence of TC without inhibitor. Surface CD23 in the presence of either of the inhibitors was quantitated by FACS analyses and compared to cells cultured without inhibitor.

### Stable transfection of CHO AAB tet off cells

50 - 80% confluent CHOAA8 cells (Clontech) grown in 6 cm dishes were transfected with 3,3 *m*g CD23b-pBEHpac18 + 6,6 *m*g pBI-ADAM9 using SuperFect essentially as recommended by the supplier (Qiagen). Clones were selected by adding 1 *m*g/ml TC + 100 *m*g/ml G418 + 5 *m*g/ml puromycin to the culture medium. Single clones were picked and transferred to 24-well plates. All clones were tested by PCR and Western blotting.

### Stimulation with PMA

Transfected CHOAA8 cells were stimulated for 30 min with 2 *m*M PMA at 37°C.

### RT-PCR analysis

RNA was isolated from 10⁶ cells using the High Pure RNA isolation kit (Roche). 5 *m*g of each of the RNA samples were reverse transcribed using M-MLV reverse transcriptase (Gibco BRL) using oligo-dT as a primer. 1 *m*l of RT mixtures were used for PCR amplification in a total volume of 25 *m*l containing 5 pmol of each primer and 0.75U of Taq polymerase (Qiagen). The primers used for amplification as well as the amplification conditions are summarized in Table 1.

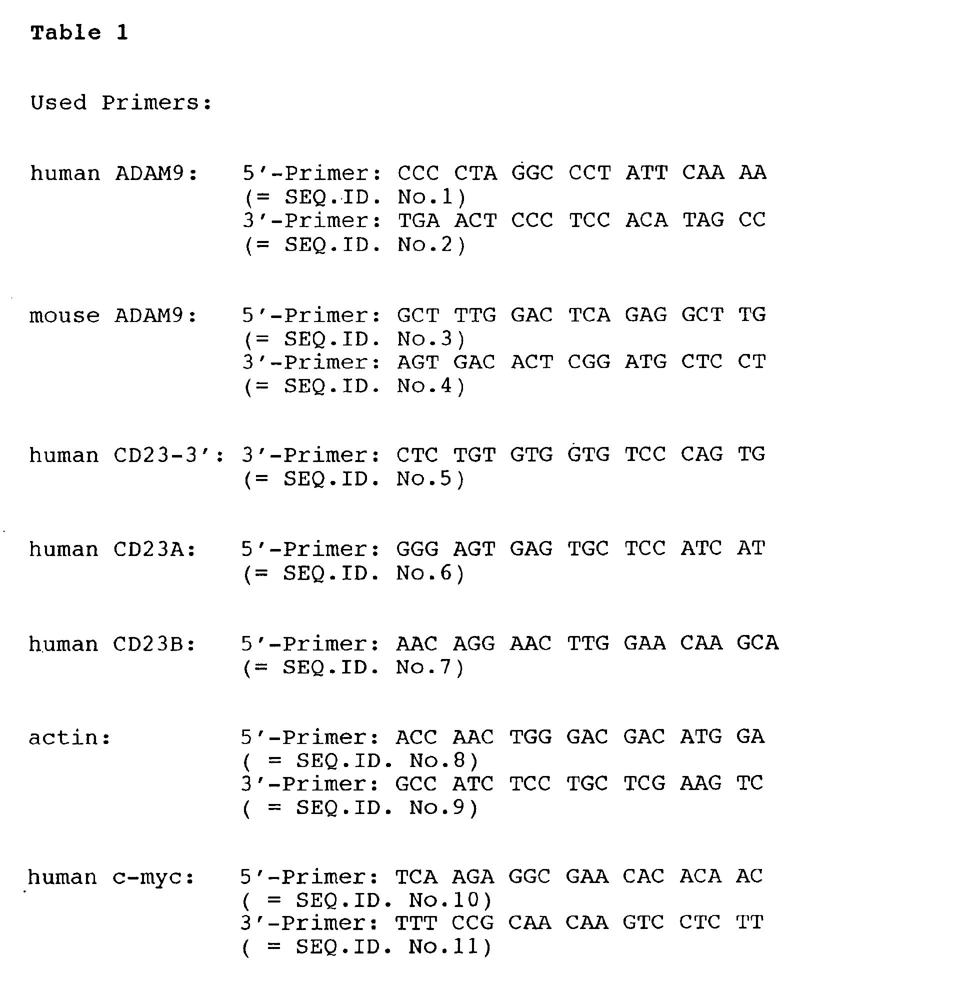

### Determination of matrix metalloproteinase activity

To determine the activity of secreted matrix metalloproteinases, P493-6 cells were cultured for 3 days (2,5 x 10⁶) with TC (1 *m*g/ml) or w/o TC. The cells were washed 3 times with PBS, resuspended in 5 ml PBS + 5 mM glucose + 10 mM HEPES and were cultured for 16 h at 37°C. Aliquots of the supernatant as well as of the washed cells were incubated with the fluorogenic Mca-peptide(7-methoxycumarin-4-yl)Acetyl-Pro-Leu-Gly-Leu-(3-[2,4-dinitrophenyl]-L-2,3-diamino-propionyl)-Ala-Arg-NH₂ (Bachem, Germany; final concentration; 200 *m*M) at 37°C in the dark (Knight et al., 1992). Subsequently, cells were pelleted and the supernatant was analyzed on a Perkin Elmer Luminescence Spectrometer L550B. The proteolytic activity towards the Gly-Leu bond was determined as fluorescence intensity following excitation at 328 nm and detection emisson at 393 nm.

### Western Blot analysis of ADAM9

2 x 10⁶ transfected or vector transfected cells were washed serum-free with cold PBS. The cell pellets were directly lyzed by adding 100 *m*l pre-boiled 2 x Laemmli buffer (Laemmli, 1970). After 5 min at 95°C the samples were centrifuged (5 min, 15000 x g). 15 *m*l aliquots were subjected to SDS PAGE (Laemmli, 1970), The separated proteins were transferred onto PVDF-membranes. The membranes were blocked using PBS + 0,25% Tween 20 (PBS/T). ADAM9 was detected using the rabbit anti-AD9Dis antibody diluted 1:125 in PBS + 0.25%Tween20 (PBS/T). After 1 h the filters were washed 3 times with PBS/T followed by an 1 h incubation with peroxidase labelled goat anti-rabbit IgG (Dianova, Hamburg) diluted 1:500 in PBS/T. After extensive washing with PBSFT bound antibody was visualized using the ECL detection system (Amersham Pharmacia Biotech).

### Generation of ADAM9 disintegrin domain (AD9Dis) specific antibodies

ADAM9 clone 2/1 (from a 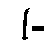 -ZAP A20 mouse B cell library, Stratagene) was used as a template to amplify the entire disintegrin domain (amino acids) using the following primers:

AD9DIS 5′end: 5′-ATT AGG ATC CGC GCC CTC CTG TGG TAA T-3′
( = SEQ.ID.No.12) and

AD9DIS 3'end: 5'-ATA TCT CGA GTC CAT TCT GAA TGA AGA C-3'.
( = SEQ.ID. No.13)

The PCR product was cloned BamH1/Xhol in frame into pGEX4T-1 (Amersham Pharmacia Biotech) and expressed as a GST fusion protein in the E.coli strain BL-21. The soluble GST-AD9Dis fusion protein was purified to homogeneity using glutathione beads essentielly as recommended by the supplier (Amersham Pharmacia Biotech) and used to immunize rabbits. The IgG fraction of the immune sera were purified on Protein A-Sepharose (Amersham Pharmacia Biotech) and GST-specific antibodies were further depleted on a BrCN-coupled GST-Sepharose 6B column. The IgG fraction was devoid of GST- and E.coli specific antibodies.

### FACS analysis of surface CD23

10⁶ cells were washed once with PBS and were then incubated with 1 *m*g FITC-labelled mouse anti-human CD23 (mAb M-L233, Pharmingen) or the respective isotype control (mouse IgG1; clone 107.3, Pharmingen) in a final volume of 50 *m*l. After 45 min the cells were washed twice with 1 ml PBS. The cells were analysed using a FACSort with CellQuest software (Becton Dickinson). CD23 analysis on B-cells that were cotransfected with the E-GFP expression plasmid was done as described above except that the anti-CD23 antibody was unlabelled. In this case, a secondary antibody (Cy3-labelled goat anti-mouse IgG, (Fab)₂ fragment, Dianova, Hamburg) was used for FACS analysis.

### Quantification of sCD23

P493-6 cells were cultured in the presence (1 *m*g/ml) or absence of TC for three days (2 x 10⁶ cells/ml). The cells were washed free of serum and were further kept serum-free in RPMI 1640 for 16 h at 37°C. 100 *m*l aliquots of the supernatants were used for the ELISA assay (BINDAZYME sCD23 immunoassay kit. The Binding Site, Birmingham, UK). All culture supernatants were tested in duplicate in at least two different dilutions. The internal standard (sCD23) was used for the calibration curve.

The attached Figures are explained by the following legends:

### Legends to Figures

### Figure 1: Shedding of CD23 in COS-7 cells by ADAM9

COS-7 cells (5-6 x 10⁵ cells /10 cm dish) were cotransfected with human CD23 in pcDNA3 (*5m*g) and either ADAM8 (in pTOP) or ADAM9, ADAM15 or ADAM19 in pcDNA3 (7.5*m*g each) using the DEAE Dextran method. Control transfections were performed with the respective expression vectors alone. 16h after transfection the cells were trypsinized and seeded into new plates. After 48h the cells were detached from the plates with ice-cold PBS+2mM CaCl₂. The cells were incubated with 1*m*g FITC-labelled anti-human CD23 or FITC-labelled mouse Ig isotype control. Surface CD23 was quantitated by FACS analysis (cells transfected with pcDNA3-CD23 and the respective vectors alone was taken as 100% CD23 expression).

### Figure 2: Shedding of human CD23 in CHOAA8 cells

RNA from CHOAA8 cell clone 1 (w/o inducible ADAM9 expression) or clone 3 (ADAM9 inducible upon withdrawal of TC)cultured in either the presence or absence of TC and PMA was reverse transcribed. Using specific primers the cDNAs for actin, ADAM9 and hCD23b was PCR amplified and separated on agarose gels. The ethidium bromide stained band intensities were quantitated by densitometric analysis and normalized to actin (Fig.2A). Cells from clone 1 and clone 3 cultured as decribed for Fig.2A were lyzed and aliquots were separated by SDS-PAGE. After blotting onto PVDF membranes ADAM9 was detected by an antibody against the disintegrin domain (AD9Dis). Bound antibody was visualized using the ECL detection system (Fig. 2B).

Cells from clone 1 and clone 3 were cultured and treated as described for Fig.2A and Fig.2B. CD23 expression of clone 1 and clone 3 kept in the presence of TC but without PMA stimulation was considered as 100% MFI (Mean Fluorescence Intensity; Fig.2C). The amount of surface CD23 was measured as described in the legend to Fig.1.

### Figure 3: CD23 expression on different human B-cell lines

The indicated human B-cell lines were incubated either with 1*m*g FITC-labelled anti-human CD23 or the respective FITC-labelled mouse Ig isotype control. The washed cells were analysed for CD23 expression by FACS analysis. CD23 expression is shown as _{D} Mean Fluorescence Intensities (_{D}MFI).

### Figure 4: Shedding of CD23 in human B cells

The human B cells P493-6, EREB2-5 and MS3 cells were cotransfected with pcDNA3-ADAM9 (or pcDNA3 as a control) and E-GFP-pIRES. 48h after transfection the cells were analysed for CD23 expression by FACS analysis (see legend to Fig. 3). The cells were gated for E-GFP expression, i.e. only transfected cells were analysed for CD23 alterations). Cells transfected with E-GFP-pIRES and vector alone was considered as 100% surface CD23 expression (Fig.4A).

MS3 cells transfected as described for Fig.4A were kept in the presence or absence of batimastat (addition of DMSO used to dissolve batimastat). Surface CD23 was analysed as described above (Fig.4B; cells kept in the presence of batimastat = 100% surface CD23).

MS3 cells were transfected with the different ADAMs as described for Fig.4A. The influence of the different ADAMs (ADAM8, ADAM9, ADAM15, ADAM19) was analyzed 48h after transfection by FACS analysis (Fig.4C).

### Figure 5: Upregulation of c-myc induces enhanced expression of ADAM9 and enhanced shedding of CD23 in human B-cells

Human P493-6 B-cells were cultured in the presence (1*m*M) or absence of TC. mRNA levels of actin, c-myc, CD23a and CD23b were analysed by RT-PCR (Fig.5A)and analysed as described in legend to Fig.2. The enhanced ADAM9 expression (mRNA level)observed in the absence of TC (100%) was normalized to actin mRNA levels obeserved in the absence or presence of TC (Fig.5A).

P493-6 cells cultured as desrcibed for Fig.5A were lyzed in detergent containing buffer and analyzed by Western blotting as described in the legend to Fig.2B (Fig.5B, insert).

Surface bound and secreted metalloproteinase activity was measured using P493-6 cells cultured in the presence (100% activity) or absence of TC using the fluorogenic Mca-peptide (Fig.5B). Surface CD23 on P493-6 cells cultured in the presence (100% mean fluorescence intensity, MFI) or absence of TC was measured by FACS analysis (see legend to Fig.3)using FITC-labelled mouse anti-CD23 (Fig.5C).

### Figure 6: Quantitative measurement of sCD23 released from P493-6 cells by shedding

P493-6 cells were kept for 3 days either in the presence (1*m*g) or absence of TC. The cells were washed free of serum and were further kept for 16h at 37°C. 100*m*l aliquots of the culture supernatants were used to measure the sCD23 concentrations using the ELISA assay kit BINDAZYME.

### Figure 7: Shedding of CD23 can be blocked by batimastat (BB-94) but not by TIMP-1 and TIMP-2

P493-6 cells were cultured for 48h either in the presence (1*m*M) or absence of TC either in the absence of any inhibitor or in the presence of 2*m*M batimastat (BB-94) or 20nM TIMP-1 or TIMP-2, respectively. The cells cultured either in the presence or absence of TC but kept in the presence of batimastat were considered as 100% surface CD23 expression. The number of CD23 molecules on P493-6 cells was measured by FACS analysis using FITC-labelled anti-CD23 and FITC-labelled mouse Ig isotype control.

### References:

Kikutani, H., Inul, S., Sato, R., Barsumian, E.L., Owakim, H., Yamasaki, K., Kaisho, T., Uchibayashi, N., Hardy, R.R., Hirano, T., Tsunasawa, S., Sakiyama, F., Suemura, M., and Kishimoto, T. (1986). Molecular structure of human lymphocyte receptor for immunoglobulin E. Cell 47, 867-885

Delespesse, G., Sarfati, M., Wu, C.Y., Fournier, S., and Letellier, M. (1992), The low affinity receptor for IgE. lmmunol. Rev. 126, 77-97

Yokota, A., Kikutani, H., Tanaka, T., Sato, R., Barsumian, E.L., Suemura, M., and Kishimoto, T. (1988). Two species of human Fcε receptor II (Fcε-RII/CD23): Tissuespecific and IL4-specific regulation of gene expression. Cell 55, 611-618

Munoz, O., Brignone, C., Grenier-Brosette, N., Bonnefoy, J.Y., and Cousin, J.L. (1998); Binding of anti-CD23 monoclonal antibody to the leucine zipper motif of FcεRII/CD23 on B cell membrane promotes its proteolytic cleavage. Evidence for an effect on the oligomer/monomer equilibrium. J.Biol.Chem. 273, 31795-31800

Gordon, J., Cairns, J.A., Liu, Y.-J, Flores-Romeo, L., MacLennan, I.C., Jansen, K.U., and Bonnefoy, J.-Y. (1991). Role of membrane and soluble CD23 in lymphocyte physiology. Monogr.Allergy 29, 156-168

Gordon, J. 1994). B-cell signalling via the C-type lectins CD23 and CD72. Immunoi. Today 15, 411-417

Law, C.L., Armitage, R.J., Viliablanca, J-G., and LeBein, T.W. (1991). Expression of interleukin-4 receptors on early human B-lineage cells. Blood 78, 703-710

Bettler, B., Maier, R., Ruegg, D., and Hofstetter, H. (1989). Binding site for IgE on the low affinity Fcε receptor (Fcε-RII/CD23) is confined to the domain homologous with animal lectins. Proc.Natl.Acad.Sci. USA 86, 7118-7122

Drickamer, K. (1988). Two distinct classes of carbohydrate-recognition domains in animal lectins. J.Biol.Chem. 263, 9557-9560

Richards, M-L. and Katz, D.H. (1990). The binding of IgE to murine FcεRll is calcium-dependent but not inhibited by carbohydrates. J. Immunol. 144, 2638-2646

Clark, E.A. (1990). CD40: a cytokine receptor in search of a ligand. Tissue Antigens 35, 33-36

Bonnefoy, J-Y., Gauchat, J.-F; Life, P., Graber, P., Mazzei, G., and Aubry, J.-P- (1996), Pairs of surface molecules involved in human IgE regulation: CD23-CD21 and CD40-CD40L. Eur. Respir. J. 9, 63-66

Punnonen, J., Aversa, G., Cocks, B.G., McKenzie, A.N., Menon, S., Zurawski, G., de Waal Malefyt, R., and de Vries, J.E. (1993). Interleukin-13 induces interleukin-4 independent IgG4 and IgE synthesis and CD23 expression in human B cells. Proc. Natl. Acad. Sci. USA 90, 3730-3734

Lebman, D.A. and Cofman, R.L. (1988). lnterleukin-4 causes isotype switching to IgE in T-cell stimulated clonal B-cell cultures. J. Exp. Med. 168, 853-862

Pene, J., Rousset, F., Briere, F., Chretien, I., Bannefoy, J.-Y., Spits, H., Yokata, T., Arai, N., Arai, K., Banchereau, J., et al. (1988). IgE production by human lymphocytes is induced by interleukin-4 and suppressed by interferon-γ and -α and prostaglandin E2. Proc. Natl. Acad. Sci. USA 85, 6880-6884

Defrance, T., Aubry, J.P., Rousset, F., Vanbervliet, B., Bonnefoy, J.-Y., Arai, N., Takebe, Y., Yokota, T., Lee, F., Arai, K., de Vries, J., and Banchereau, J. (1987). Human recombinant interleukin-4 induces Fcε receptors (CD23) on normal B-lymphocytes- J. Exp. Med. 165, 1459-1467

Bonnefoy, J.-Y., Plater-Zyberk, C., Lecoanet-Henchoz, S., Gauchat, J.-F., Aubry, J.-P., and Graber, P, (1996). A new role of CD23 in inflammation. Immunol. Today 17, 418-420

Lecoanet-Henchoz, S., Gauchat, J.-F., Aubry, J.-P., Graber, P., Life, P., Paul-Eugene, N., Ferrua, B., Corbi, A.L., Dugas, B., Plater-Zyberk, C. and Bonnefoy, J.-Y. (1995). CD23 regulates monocyte activation through a novel interaction with the adhesion molecules CD 11b-CD 18 and CD 11c-CD 18. Immunity 3, 119-125

Hellen, E.A., Rowlands, D.C., Hansel, T.T., Ktas, G.D., and Crocker, J. (1991) Immunohistochemical demonstration of CD23 expression on lymphocytes in rheumatoid synovitis. J. Clin. Pathol. 44, 293-296

Bansal, A., Roberts, T., Hay, E.M., Kay, R., and Pumphrey, R.S. (1992). Soluble CD23 levels are elevated in the serum of patients with primary Sjögren's syndrome and systemic lupus erythematosus. Clin. Exp. Immunol. 89, 452-455

Yano, N., Mlyazaki, M., Endoh, M., Kuramoto, T., Eguchi, K., Yagame, M., Nomoto, Y., and Sakai, H. (1992). Increase of CD23-positive cells in peripheral blood from patients with IgA nephropathy and non-IgA proliferative glomerulonephritis. Nephron 60, 404-41 0

Kleinau, S., Martinsson, P., Gustavsson, S. and Heyman, B. (1999). Importance of CD23 for collagen-induced arthritis: delayed onset and reduced severity in CD23-deficient mice. J. Immunol. 162, 4266-4270

Letellier, M., Nakajima, T-, Pulido-Cejudo, T., Hofstetter, H., and Delespesse, G. (1990). Mechanism of formation of human IgE-binding factors (soluble CD23): III. Evidence for a receptor (Fc epsilon RII)-associated proteolytic activity. J. Exp. Med. 172, 693-700

Letellier, M., Sarfati, M., and Delespesse, G, (1 989). Mechanisms of formation of IgE binding factors (soluble CD23), I, Fc epsilon RII bearing B cells generate IgE binding factors of different molecular weights. Mol. Immunol. 26, 1105-1112

Delespesse, G., Suter, U., Mossalayi, D., Bettler, B., Sarfati, M., Hofstetter, H., Kilcherr, E., Debre, P., and Dalloul, A. (1991), Expression, structure and function of the CD23 antigen. Adv. Immunol. 49, 149-191

Swendeman, S. and Thorely-Lawson, D-A. (1 987). The activation antigen Blast-2, when shed, is an autocrine growth factor for normal and transformed B cells. EMBO J. 6, 1637-1642

Mossalayi, D., Lecron, J.-C., Dalloul, A-H., Sarfati, M., Bertho, J.-M., Hofstetter, H., Delespesse, G., and Debre, P. (1990). Soluble CD23 (FcεRII) and interleukin 1 synergistically induce early human thymocyte maturation. J. Exp. Med. 171, 959-964

Liu, Y.-J-, Cairns, J.A., Holder, M.J., Abbot, S.D., Jansen, K-U., Bonnefoy, J.Y-, Gordon, J., and MacLennan, I.C.M. (1991). Recombinant 25-kDa CD23 and interleukin-1α promote the survival of germinal centre B cells: Evidence for birfurcation in the development of centrocytes rescued from apoptosis. Eur. J. Immunol. 21, 1107-1114

Liu, Y.-J, Mason, D.Y., Johnson, G.D., Abbot, S., Gregory, C.D., Hardie, D.L., Gordon, J., and MacLennan, I.C.M. (1991). Germinal centre cells express bcl-2 protein after activation by signals which prevent their entry into apoptosis. Eur. J. Immunol. 21, 1905-1910

Hermann, P., Armant, M., Rubio, M., Ishihara, H., Ulrich, D., Caspary, R,G., Lindberg, F.P., Armitage, R., Maliszewski, C., Delespesse, G., and M. Sarfati (1999). The vitronectin receptor and its associated CD47 molecule mediates proinflammatory cytokine synthesis in human monocytes by interaction with soluble CD23. J. Cell Biol. 144, 767-775

Christie, G., Barton, A., Bolognese, B., Buckle, D.R., Cook, R.M., Hansbury, M.J., Harper, G.P., Marshall, L.A., McCord, M.E., Moulder, K., Murdock, P.R., Seal, S.M., Spackman, V.M., Weston, B.J., and Mayer, R.J. (1997). Inhibition of sCD23 and immunoglobulin E release from human B cells by a metalloproteinase inhibitor, Gl 129471. Eur. J. Immunol. 27, 3228-3235

Munoz, 0., Brignone, C., Grenier-Brossette, N., Bonnefoy, J.-Y., and Cousin, J.L(1998). Binding of anti-CD23 monoclonal antibody to the leucine zipper motif of FcepsilonRII/CD23 on B cell membrane promotes its proteolytic cleavage. Evidence for an effect on the oligomer/monomer equilibrium. J. Biol-Chem. 273, 31795-31800

Bailey, S., Bolognese, B., Buckle, D.R., Faller, A., Jackson, S., Lowis-Flamberg, P., McCord, M., Mayer, R., Marshall, L.A., and Smith, D.G. (1998). Hydroxamate-based inhibitors of low affinity IgE receptor (CD23) processing. Bioorg. Med. Chem. Lett. 8, 2328

Bailey, S., Bolognese, B., Buckle, D.R., Faller, A., Jackson, S., Louis-Flamberg, P., McCord, M., Mayer, R., Marshall, L.A., and Smith, D.G. (1998). Selective inhibition of low affinity IgE receptor (CD23) processing. Bioorg. Med. Chem. Lett. 8, 29-34

Knight, C.G., Willenbrock, F., and Murphy, G. (1992). A novel coumarin-labelled peptide for sensitive continous assays of the matrix metalloproteinases. FEBS Lett. 296, 263-266

Marolewski, A.E., Buckle, D.R. Christie, G., Earnshaw, D.L., Flamberg, P.L. Marshall, L-A., Smith, D.,G., and Mayer, R.J. (1998). CD23 (FcRII) release from cell membranes is mediated by a membrane-bound metalloproteinase. Biochem. J. 333, 573-579

Hooper, N.M., Karran, E.H., and Turner, A.J. (1997). Membrane protein secretases. Biochem. J. 321, 265-279

Black, R., Rauch, C.T., Koziosky, C.J., Peschon, J.J., Slack, J.L. Wolfson, M.F., Castner, B.J., Stocking, K.L., Reddy, P., Srinivasan, S, et al. (1997). Nature 385, 729733

Moss, M.L., Jin, S.-L.C., Milla, M.E., Burkhart, W., Cartner, H.L., Chen, W.-J., Clay, W.C., Didsbury, J.R., Hassler, D., Hoffman, C.R. et al. (1997). Cloning a disintegrin metalloproteinase that processes precursor tumor-necrosis factor-α. Nature 385, 733-736

Blobel, C.P. and White, J.M. (1992). Structure, function and evolutionary relationship Of proteins containing a disintegrin domain. Curr. Opin. Cell Biol. 4, 760-765

Black, R.A. and White, J.M. (1998). ADAMS: focus on the protease domain. Curr. Opin, Cell Biol. 10, 654-659

Schlömdorff, J. and Blobel, C.P. (1999). Metalloprotease-disintegrins: modular proteins capable of promoting cell-cell interactions and triggering signals by protein-ectodomain shedding. J. Cell Sci. 112, 3603-3617

Bode, W., Gomis-Ruth, F.X., and Stockler, W. (1993). Astacins, serralysins, snake venom and matrix metalloproteinases exhibit identical zinc-binding environments (HEXXHXXGXXH and Met-turn) and topologies and should be grouped into a common family, the'metzincins'. FEBS Lett, 331, 134-140

Weskamp, G., Krätzschmar, J.R., Reid, M., and Blobel, C.P. (1996). MDC9, a widely expressed cellular disintegrin containing cytoplasmic SH3 ligand domains. J. Cell Biol. 132, 717-726

Aruffo, A, and Seed, B. (1987). Molecular cloning of a CD28 CDNA by a high-efficiency COS cell expression system. Proc. Natl. Acad. Sci. USA 84, 8573-8577

Gossen, M. and Bujard, H. (1992). Tight control of gene expression in mammalian cells by tetracycline-responsive promotors. Proc. Natl. Acad. Sci. USA 89, 5547-5551

Staege, M.S., Schneider, J., Eulitz, M., Scholz, S., Bornkamm, G.W., Wölfel, T., Reske-Kunz, A.B. (2000). Consequences of antigen self-presentation of tumor specific T-cells. Immunobiol. 201, 332-346

Schuhmacher, M., Staege, M.S., Pajic, A., Polack, A., Weidle, U.H., Bornkamm, G.W., Eick, D., and Kohlhuber, F. (1999).

Control of cell growth by c-myc in the absence of cell division. Curr, Biol. 9, 1255-1258

Kempkes, B., Spitkovsky, D., Jansen-Dürr, P., Eilwart, J.W. Kremmer, E., Delecluse, H.J., et al. (1995). B-Cell proliferation and induction of early Gl-regulating proteins by Epstein-Barr virus mutants conditional for EBNA2. EMBO J. 14, 88-96

Artelt, P., Morelle, C., Ausmeier, M., Fitzek, M., and Hauser, H. (1988). Vectors for efficient expression in mammalian fibroblastoid and lymphoid cells via transfection or infection. Gene 68, 213-219

Laemmli, U.K. (1970). Cleavage of structural proteins during the assembly of the head of bacteriophage T4. Nature, 227, 680-685

## Claims

1. A pharmaceutical composition for the treatment or prophylaxis of disorders in which the overproduction of s-CD23 is implicated, which comprises an inhibitor for the formation of human soluble CD23, **characterised in that** the inhibitor is a compound which selectively decreases or blocks the activity of the metalloprotease ADAM9 which otherwise mediates the shedding of s-CD23 in human B-cell lines.

2. A pharmaceutical composition as claimed in claim 1, **characterised in that** the inhibitor is a monoclonal or polyclonal antibody which is selectively directed against the metalloprotease ADAM9.

3. A pharmaceutical composition as claimed in claims 1 and 2, **characterised in that** it comprises an antisense oligonucleotide which is specific for c-myc.

4. An antibody which selectively binds to the metallo-protease ADAM9.

5. A monoclonal antibody as claimed in claim 4.

6. A humanised antibody as claimed in claims 4 and 5.

7. An antibody as claimed in claims 4 - 6 for use in human therapy.

8. Use of an antibody as claimed in claims 4 - 7 in the manufacture of a medicament for the treatment of disorders which are associated with excess production of soluble human CD23.

9. Use of an an antisense oligonucleotide which is specific for c-myc in the manufacture of a medicament for the treatment of disorders which are associated with an excess production of soluble human CD23.

10. A method of treating a patient suffering from disorders in which the overproduction of s-CD23 is implicated comprising administering to said patient a therapeutically effective amount of an inhibitor which is specific for the metalloprotease ADAM9.

11. A method of treating a patient as claimed in claim 10 comprising administering to said patient a therapeutically effective amount of an antibody as claimed in claims 4 - 7.

12. A method of treating a patient as claimed in claim 10 comprising administering to said patient a therapeutically effective amount of an oligonucleotide which is specific for c-myc.


SEQUENCE LISTING

<110> Prof. Dr. Frey, Juergen

<120> Inhibitors for the formation of soluble human CD 23

<130> F53P1EP

<140>
<141>

<160> 13

<170> PatentIn Ver. 2.1

<210> 1
<211> 20
<212> DNA
<213> Homo sapiens

<400> 1
cccctaggcc ctattcaaaa                                              20

<210> 2
<211> 20
<212> DNA
<213> Homo sapiens

<400> 2
tgaactccct ccacatagcc                                              20

<210> 3
<211> 20
<212> DNA
<213> Mus musculus

<400> 3
gctttggact cagaggcttg                                                    20

<210> 4
<211> 20
<212> DNA
<213> Mus musculus

<400> 4
agtgacactc ggatgctcct                                                    20

<210> 5
<211> 20
<212> DNA
<213> Homo sapiens

<400> 5
ctctgtgtgg tgtcccagtg                                                    20

<210> 6
<211> 20
<212> DNA
<213> Homo sapiens

<400> 6
gggagtgagt gctccatcat                                                    20

<210> 7
<211> 21
<212> DNA
<213> Homo sapiens

<400> 7
aacaggaact tggaacaagc a                                                21

<210> 8
<211> 20
<212> DNA
<213> Homo sapiens

<400> 8
accaactggg acgacatgga                                                  20

<210> 9
<211> 20
<212> DNA
<213> Homo sapiens

<400> 9
gccatctcct gctcgaagtc                                                  20

<210> 10
<211> 20
<212> DNA
<213> Homo sapiens

<400> 10
tcaagaggcg aacacacaac                                                  20

<210> 11
<211> 20
<212> DNA
<213> Homo sapiens

<400> 11
tttccgcaac aagtcctctt                                                    20

<210> 12
<211> 28
<212> DNA
<213> Mus musculus

<400> 12
attaggatcc gcgccctcct gtggtaat                                           28

<210> 13
<211> 28
<212> DNA
<213> Mus musculus

<400> 13
atatctcgag tccattctga atgaagac                                           28